# EUROPEAN PATENT APPLICATION

(11) **EP 4 772 168 A1**
(43) Date of publication of application: **08.07.2026**
(21) Application number: 24860418.3
(22) Date of filing: 28.08.2024
(51) Int. Cl.: A61K 9/00, A61K 47/10, A61K 9/08, A61K 31/4725, A61P 27/02, A61P 27/04

(54) **EYE DROP COMPOSITION COMPRISING LIFITEGRAST**

(30) Priority: 01.09.2023 KR 20230116348
(71) Applicant: YS Life Science Co., Ltd., Hwaseong-si, Gyeonggi-do 18581 (KR)
(72) Inventor: PARK, Jong Ho, Suwon-si, Gyeonggi-do 16226 (KR); BAEK, Areum, Suwon-si, Gyeonggi-do 16674 (KR); CHUNG, Young Sik, Yongin-si, Gyeonggi-do 17066 (KR); CHUN, Myung Hee, Suwon-si, Gyeonggi-do 16325 (KR); LEE, Kee Young, Seoul 06642 (KR)
(74) Representative: V.O.
(86) International application number: PCT/KR2024/012898
(87) International publication number: WO 2025/048498

(57) **Abstract**

The present invention provides an ophthalmic composition comprising lifitegrast or a pharmaceutically acceptable salt thereof, and propylene glycol as an anti-degradation agent. The ophthalmic composition according to the present invention exhibits excellent stability by inhibiting the degradation of active ingredients without side effects caused by antioxidants.

## Description

### [Technical Field]

The present invention relates to an ophthalmic composition comprising lifitegrast. More particularly, the present invention relates to an ophthalmic composition comprising lifitegrast having excellent stability without side effects from antioxidants.

### [Background Art]

Tears act as a lubricant, facilitating smooth eye movement. Tears consist of a mucous layer, an aqueous layer, and a lipid layer. The mucous layer adheres tears to the eye surface, ensuring even distribution of tears. The aqueous layer removes impurities and cleans the eye surface. The lipid layer inhibits tear evaporation. Dry eye syndrome occurs when the balance of these layers is disrupted due to aging or external factors like screen use, medication side effects, or contact lens wear.

Dry eye syndrome is a common condition with symptoms including eye redness, swelling, foreign body sensation, fatigue, burning, and itching. In severe cases, it can affect vision. Artificial tears containing active ingredients such as lifitegrast, diquafosol, sodium carboxymethylcellulose, cyclosporine, and sodium hyaluronate are used to treat dry eye syndrome.

Among these, lifitegrast is an integrin receptor antagonist used in dry eye treatments. It works by binding to lymphocyte function-associated antigen-1 (LFA-1) and inhibiting the interaction with intercellular adhesion molecule-1 (ICAM-1), thereby reducing inflammation.

Most commercially available lifitegrast-containing dry eye treatments contain antioxidants to inhibit the oxidative degradation of the active ingredient in the eye drops. However, these antioxidants can cause toxicity to ocular cells and may induce discomfort and inflammation in the eyes of patients with severe dry eye.

For example, Korean Patent Publication No. 10-2015-0100763 discloses the use of thiosulfate salts as antioxidants to prevent the oxidative degradation of lifitegrast. However, thiosulfate salts can cause irritation such as redness, itching, and conjunctivitis when instilled.

Therefore, there is a need for the development of an ophthalmic composition comprising lifitegrast with excellent stability that inhibits the degradation of the active ingredient without side effects caused by antioxidants.

### [Disclosure]

### [Technical Problem]

An object of the present invention is to provide an ophthalmic composition comprising lifitegrast with excellent stability that inhibits the degradation of the active ingredient without side effects caused by antioxidants.

### [Technical Solution]

An aspect of the present invention relates to an ophthalmic composition comprising lifitegrast or a pharmaceutically acceptable salt thereof, and propylene glycol as an anti-degradation agent.

In one embodiment of the present invention, the propylene glycol may be included at a concentration of 0.2 to 5% (w/v).

The ophthalmic composition according to one embodiment of the present invention may further comprise one or more additives selected from a pH adjuster, a buffer, and an isotonic agent.

In one embodiment of the present invention, the pH adjuster may be one or more selected from the group consisting of lactic acid, hydrochloric acid, phosphoric acid, citric acid, acetic acid, sodium hydroxide, potassium hydroxide, sodium carbonate, and sodium bicarbonate.

In one embodiment of the present invention, the buffer may be one or more selected from the group consisting of phosphoric acid, phosphate, carbonate, bicarbonate, tris(hydroxymethyl)aminomethane (TRIS), lactic acid, lactate, citric acid, citrate, boric acid, borate, acetic acid, and acetate.

In one embodiment of the present invention, the isotonic agent may be one or more selected from the group consisting of glycerin, sodium chloride, potassium nitrate, potassium chloride, calcium chloride, magnesium chloride, trehalose, maltose, sucrose, mannitol, sorbitol, and boric acid.

The ophthalmic composition according to one embodiment of the present invention may be in the form of an aqueous solution.

The ophthalmic composition according to one embodiment of the present invention may have a pH within the range of 7 to 8.

The ophthalmic composition according to one embodiment of the present invention may contain no antioxidant.

In one embodiment of the present invention, the antioxidant may be one or more of dibutylhydroxytoluene, ascorbic acid, phenolic acid, sorbic acid, sodium bisulfite, sodium metabisulfite, acetylcysteine, sodium thiosulfate, ethylenediaminetetraacetic acid (EDTA), sodium nitrite, ascorbyl stearate, ascorbyl palmitate, alpha-thio-glycerol, erythorbic acid, cysteine hydrochloride, citric acid, tocopherol, tocopherol acetate, soy lecithin, sodium thioglycolate, butylated hydroxyanisole, propyl gallate, uric acid, melatonin, and thiourea.

The ophthalmic composition according to one embodiment of the present invention may be for treating dry eye syndrome.

### [Advantageous Effects]

The ophthalmic composition according to the present invention exhibits excellent stability by inhibiting the degradation of active ingredients without side effects caused by antioxidants, as it contains propylene glycol as an anti-degradation agent and contains no antioxidant.

### [Description of Drawings]

FIG. 1 is a graph showing the amount of total degradation products generated after storing the ophthalmic compositions of Example 1 and Comparative Examples 1 to 9 at 40°C for 8 weeks.
FIG. 2 is a graph showing the amount of total degradation products generated after storing the ophthalmic compositions of Example 1 and Comparative Examples 1 to 9 at 60°C for 4 weeks.
FIG. 3 is a photograph showing the appearance of the ophthalmic compositions of Examples 2 to 4 and Comparative Example 1 after storage for 24 weeks at 40±2°C and 25% RH.
FIG. 4 is a graph showing the amount of total degradation products generated after storing the ophthalmic compositions of Examples 1 to 4 and Comparative Examples 1, 8, and 9 at 40±2°C and 25% RH for 24 weeks.
FIG. 5 is a graph showing the amount of total degradation products generated after storing the ophthalmic compositions of Examples 1 to 4 and Comparative Examples 1, 8, and 9 at 50±2°C and 20% RH for 4 weeks.

### [Best Mode]

Hereinafter, the present invention will be described in more detail.

One embodiment of the present invention relates to an ophthalmic composition comprising lifitegrast or a pharmaceutically acceptable salt thereof, and propylene glycol as an anti-degradation agent.

In one embodiment of the present invention, the lifitegrast is a compound of the following formula (1), which is the active pharmaceutical ingredient (API) of Xiidra^{®}, a dry eye treatment agent.

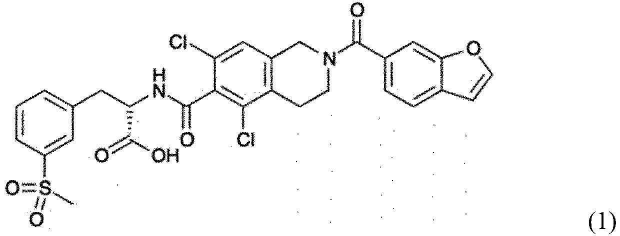

In one embodiment of the present invention, the pharmaceutically acceptable salt of the lifitegrast may be any salt commonly used in the art without limitation, including, for example, both non-toxic inorganic and organic salts, and may be base addition salts. Such base addition salts include alkali metal or alkaline earth metal salts such as lithium, sodium, potassium, magnesium and calcium; ammonium salts and quaternary ammonium salts such as tetramethylammonium; amine salts such as methylamine, dimethylamine, diethylamine, trimethylamine, triethylamine, ethylamine, isopropylamine, methanolamine, ethanolamine, dimethanolamine, diethanolamine, 2-dimethylaminoethanol, 2-diethylaminoethanol, dicyclohexylamine, lysine, arginine, histidine, caffeine, procaine, N,N-dibenzylethylenediamine, chloroprocaine, hydrabamine, choline, betaine, ethylenediamine, ethylenedianiline, glucosamine, methylglucamine, theobromine, purine, piperazine, piperidine, N-ethylpiperidine and meglumine; polyamine resins, etc.

The content of the lifitegrast or pharmaceutically acceptable salt thereof may be appropriately selected within therapeutically effective concentration ranges, specifically ranging from 0.01 to 10% (w/v) based on the total ophthalmic composition.

In one embodiment of the present invention, the propylene glycol acts as an anti-degradation agent in the ophthalmic composition, minimizing the generation of degradation products from the active ingredient to maintain the appearance of the composition and ensure stability.

The propylene glycol may be included at a concentration of 0.2 to 5% (w/v), preferably 0.5 to 3% (w/v). If the concentration of the propylene glycol is below this range, the anti-degradation effect may be reduced. If it exceeds this range, it may cause contact dermatitis or exhibit irritancy.

The ophthalmic composition according to one embodiment of the present invention may further include one or more additives selected from pH adjusters, buffers, and isotonic agents.

The pH adjuster may be one or more selected from the group consisting of lactic acid, hydrochloric acid, phosphoric acid, citric acid, acetic acid, sodium hydroxide, potassium hydroxide, sodium carbonate, and sodium bicarbonate, and may be particularly hydrochloric acid, sodium hydroxide, or a combination thereof for the stability of the ophthalmic composition.

The buffer may be one or more selected from the group consisting of phosphoric acid, phosphate, carbonate, bicarbonate, tris(hydroxymethyl)aminomethane (TRIS), lactic acid, lactate, citric acid, citrate, boric acid, borate, acetic acid, and acetate, and may be a phosphate, particularly for the stability of the ophthalmic composition. As the phosphate, disodium hydrogen phosphate or a hydrate thereof, sodium dihydrogen phosphate or a hydrate thereof, etc. may be used.

The isotonic agent may be one or more selected from the group consisting of glycerin, sodium chloride, potassium nitrate, potassium chloride, calcium chloride, magnesium chloride, trehalose, maltose, sucrose, mannitol, sorbitol, and boric acid, and may be sodium chloride, particularly for the stability of the ophthalmic composition.

The ophthalmic composition according to one embodiment of the present invention may be in the form of an aqueous solution. In this case, the aqueous medium may be water for injection, sterile purified water, physiological saline, etc.

The ophthalmic composition according to one embodiment of the present invention may have a pH within the range of 7 to 8. When the pH is adjusted to the above range, the stability of the ophthalmic composition is excellent and irritation upon instillation can be reduced.

The ophthalmic composition according to one embodiment of the present invention may not contain antioxidants that are commonly used for the purpose of inhibiting the degradation of the active substance and preventing oxidation.

If the ophthalmic composition according to one embodiment of the present invention contains an antioxidant, its stability may be reduced.

The ophthalmic composition according to one embodiment of the present invention may also prevent side effects such as redness, itching, and conjunctivitis that may be induced by the antioxidant during instillation, by not containing an antioxidant.

Specifically, the antioxidant may include dibutylhydroxytoluene, ascorbic acid, phenolic acid, sorbic acid, sodium bisulfite, sodium metabisulfite, acetylcysteine, sodium thiosulfate, ethylenediaminetetraacetic acid (EDTA), sodium nitrite, ascorbyl stearate, ascorbyl palmitate, alpha-thio-glycerol, erythorbic acid, cysteine hydrochloride, citric acid, tocopherol, tocopherol acetate, soy lecithin, sodium thioglycolate, butylated hydroxyanisole, propyl gallate, uric acid, melatonin, thiourea, or salts or combinations thereof, for example.

Furthermore, the ophthalmic composition according to one embodiment of the present invention may substantially not contain a preservative.

The ophthalmic composition according to one embodiment of the present invention can prevent side effects such as irritation, inflammation, and cytotoxicity that may be induced by the preservative, by not containing a preservative.

Specifically, the preservatives may include quaternary ammonium salts such as benzalkonium chloride, benzethonium chloride, cetalkonium chloride, and polyquaternium-1 (e.g., polyquad); guanidine compounds such as PHMB and chlorhexidine; alcohol compounds such as chlorobutanol and benzyl alcohol; mercury compounds such as thimerosal, phenylmercury acetate, and phenylmercury nitrate; para-hydroxybenzoic acid ester compounds such as methylparaben and propylparaben; sorbic acid compounds such as potassium sorbate, for example.

The ophthalmic composition according to one embodiment of the present invention contains propylene glycol as an anti-degradation agent and substantially does not contain antioxidants and/or preservatives, thereby maintaining the appearance of the ophthalmic composition even after long-term storage without side effects caused by antioxidants and/or preservatives and minimizing the generation of degradation products.

The ophthalmic composition according to one embodiment of the present invention may be used for treating dry eye syndrome by including lifitegrast or a pharmaceutically acceptable salt thereof as an active ingredient.

Hereinafter, the present invention will be described more specifically by means of examples, comparative examples, and experimental examples. These examples, comparative examples, and experimental examples are intended to illustrate the present invention only, and it is obvious to those skilled in the art that the scope of the present invention is not limited to them.

### Example 1 and Comparative Examples 1 to 9: Preparation of ophthalmic compositions according to the type of anti-degradation agent

The ophthalmic compositions were prepared according to the following method using the compositions shown in Tables 1 and 2 (units: %, w/v).

While stirring the water for injection, the isotonic agent, buffer, and active ingredient lifitegrast were added and dissolved. Each anti-degradation agent was then added and dissolved, and the pH was adjusted to 7 to 8 using a pH adjuster. The ophthalmic composition was prepared by filtering through a sterile filter of 0.2 µm or less.

For Example 1, dibutylhydroxytoluene, an antioxidant, was added along with the anti-degradation agent, and polysorbate 80, a surfactant, was further added to dissolve it.

**[Table 1]**

| Category | Ingredient name | Comparat ive Example 1 | Comparat ive Example 2 | Comparat ive Example 3 | Comparat ive Example 4 | Compara tive Example 5 |
|---|---|---|---|---|---|---|
| Active ingredient | Lifitegrast | 5.0 | 5.0 | 5.0 | 5.0 | 5.0 |
| Anti-degradation agent | Sodium bisulfite | | 0.2 | | | |
| | TPGS | | | 0.5 | | |
| | Ascorbic acid | | | | 0.3 | |
| | Sodium Metabisulfite | | | | | 0.4 |
| Isotonic agent | Sodium chloride | 0.305 | 0.305 | 0.305 | 0.305 | 0.305 |
| Buffer | Anhydrous disodium hydrogen phosphate | 0.355 | 0.355 | 0.355 | 0.355 | 0.355 |
| PH adjuster | Sodium hydroxide | Appropria te amount | Appropria te amount | Appropri ate amount | Appropria te amount | Appropri ate amount |
| | Hydrochloric acid | Appropria te amount | Appropria te amount | Appropri ate amount | Appropria te amount | Appropri ate amount |
| Water for injection | | Residual amount | Residual amount | Residual amount | Residual amount | Residual amount |

**[Table 2]**

| Category | Ingredient name | Comparat ive Example 6 | Comparat ive Example 7 | Comparat ive Example 8 | Comparat ive Example 9 | Example 1 |
|---|---|---|---|---|---|---|
| Active ingredient | Lifitegrast | 5.0 | 5.0 | 5.0 | 5.0 | 5.0 |
| Anti-degradation agent | Phosphoric acid | 0.228 | | | | |
| | Sodium sulfite | | 0.2 | | | |
| | Potassium sorbate | | | 0.47 | | |
| | Hyaluronic acid | | | | 0.3 | |
| | Propylene glycol | | | | | 3 |
| Antioxidant | Dibutvlhvdroxytoluene | | | | | 0.005 |
| Surfactant | Polysorbate 80 | | | | | 0.25 |
| Isotonic agent | Sodium chloride | 0.305 | 0.305 | 0.305 | 0.305 | 0.305 |
| Buffer | Anhydrous disodium hydrogen phosphate | 0.355 | 0.355 | 0.355 | 0.355 | 0.355 |
| PH adjuster | Sodium hydroxide | Appropri ate amount | Appropri ate amount | Appropri ate amount | Appropri ate amount | Appropri ate amount |
| | Hydrochloric acid | Appropri ate amount | Appropri ate amount | Appropri ate amount | Appropri ate amount | Appropri ate amount |
| Water for injection | | Residual amount | Residual amount | Residual amount | Residual amount | Residual amount |

### Experimental Example 1: Stability of ophthalmic compositions according to the type of anti-degradation agent

The ophthalmic compositions of the above Examples and Comparative Examples were placed in glass bottles, tightly sealed, and stored at 40°C for 8 weeks and at 60°C for 4 weeks.

Subsequently, the amount of degradation products formed was measured using liquid chromatography.

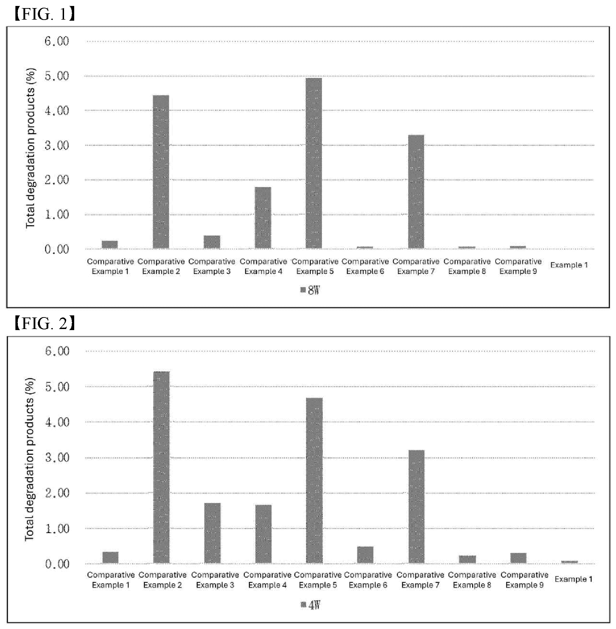

The results are shown in Tables 3 and 4 below, and FIGS. 1 and 2.

FIG. 1 is a graph showing the amount of total degradation products generated after storing the ophthalmic compositions of Example 1 and Comparative Examples 1 to 9 at 40°C for 8 weeks. FIG. 2 is a graph showing the amount of total degradation products generated after storing the ophthalmic compositions of Example 1 and Comparative Examples 1 to 9 at 60°C for 4 weeks.

**[Table 3]**

| | Storage conditions | Item | Initial (%) | 8 weeks (%) |
|---|---|---|---|---|
| Comparative Example 1 | 40°C | Individual degradation products | Not detected | 0.17 |
| | | Total degradation products | Not detected | 0.23 |
| Comparative Example 2 | 40°C | Individual degradation products | 0.14 | 2.56 |
| | | Total degradation products | 0.19 | 4.43 |
| Comparative Example 3 | 40°C | Individual degradation products | Not detected | 0.22 |
| | | Total degradation products | Not detected | 0.39 |
| Comparative Example 4 | 40°C | Individual degradation products | 0.06 | 0.56 |
| | | Total degradation products | 0.06 | 1.78 |
| Comparative Example 5 | 40°C | Individual degradation products | 0.18 | 2.59 |
| | | Total degradation products | 0.23 | 4.94 |
| Comparative Example 6 | 40°C | Individual degradation products | Not detected | 0.07 |
| | | Total degradation products | Not detected | 0.07 |
| Comparative Example 7 | 40°C | Individual degradation products | 0.10 | 1.99 |
| | | Total degradation products | 0.10 | 3.29 |
| Comparative Example 8 | 40°C | Individual degradation products | Not detected | 0.07 |
| | | Total degradation products | Not detected | 0.07 |
| Comparative Example 9 | 40°C | Individual degradation products | Not detected | 0.08 |
| | | Total degradation products | Not detected | 0.08 |
| Example 1 | 40°C | Individual degradation products | Not detected | Not detected |
| | | Total degradation products | Not detected | Not detected |

**[Table 4]**

| | Storage conditions | Item | Initial (%) | 4 weeks (%) |
|---|---|---|---|---|
| Comparative Example 1 | 60°C | Individual degradation products | Not detected | 0.22 |
| | | Total degradation products | Not detected | 0.33 |
| Comparative Example 2 | 60°C | Individual degradation products | 0.14 | 2.79 |
| | | Total degradation products | 0.19 | 5.42 |
| Comparative Example 3 | 60°C | Individual degradation products | Not detected | 0.77 |
| | | Total degradation products | Not detected | 1.71 |
| Comparative Example 4 | 60°C | Individual degradation products | 0.06 | 0.60 |
| | | Total degradation products | 0.06 | 1.65 |
| Comparative Example 5 | 60°C | Individual degradation products | 0.18 | 2.27 |
| | | Total degradation products | 0.23 | 4.67 |
| Comparative Example 6 | 60°C | Individual degradation products | Not detected | 0.27 |
| | | Total degradation products | Not detected | 0.48 |
| Comparative Example 7 | 60°C | Individual degradation products | 0.10 | 1.58 |
| | | Total degradation products | 0.10 | 3.20 |
| Comparative Example 8 | 60°C | Individual degradation products | Not detected | 0.17 |
| | | Total degradation products | Not detected | 0.23 |
| Comparative Example 9 | 60°C | Individual degradation products | Not detected | 0.18 |
| | | Total degradation products | Not detected | 0.30 |
| Example 1 | 60°C | Individual degradation products | Not detected | 0.08 |
| | | Total degradation products | Not detected | 0.08 |

From Tables 3 and 4 and FIGS. 1 and 2, it can be seen that the ophthalmic composition of Example 1, which contains propylene glycol as an anti-degradation agent, produces fewer degradation products compared to the ophthalmic compositions of Comparative Examples 1 to 9, which either do not contain an anti-degradation agent or contain a different anti-degradation agent instead of propylene glycol.

### Examples 2 to 4: Preparation of antioxidant-free ophthalmic compositions

Ophthalmic compositions were prepared according to the method of Example 1 using the compositions shown in Table 5 below (units: %, w/v).

**[Table 5]**

| Category | Ingredient name | Example 2 | Example 3 | Example 4 |
|---|---|---|---|---|
| Active ingredient | Lifitegrast | 5.0 | 5.0 | 5.0 |
| Anti-degradation agent | Propylene glycol | 3 | 0.75 | 3 |
| Surfactant | Polysorbate 80 | | | 0.25 |
| Isotonic agent | Sodium chloride | 0.305 | 0.305 | 0.305 |
| Buffer | Anhydrous disodium hydrogen phosphate | 0.355 | 0.355 | 0.355 |
| PH adjuster | Sodium hydroxide | Appropriate amount | Appropriate amount | Appropriate amount |
| | Hydrochloric acid | Appropriate amount | Appropriate amount | Appropriate amount |
| Water for injection | | Residual amount | Residual amount | Residual amount |

### Experimental Example 2: Stability of ophthalmic compositions with and without antioxidants

The ophthalmic compositions from the above examples were packaged in single-dose LDPE containers and stored for 24 weeks at 40±2°C and 25% RH, and for 4 weeks at 50±2°C and 20% RH, respectively.

Subsequently, the appearance of the ophthalmic compositions was visually inspected, and the amount of degradation products generated was measured using liquid chromatography.

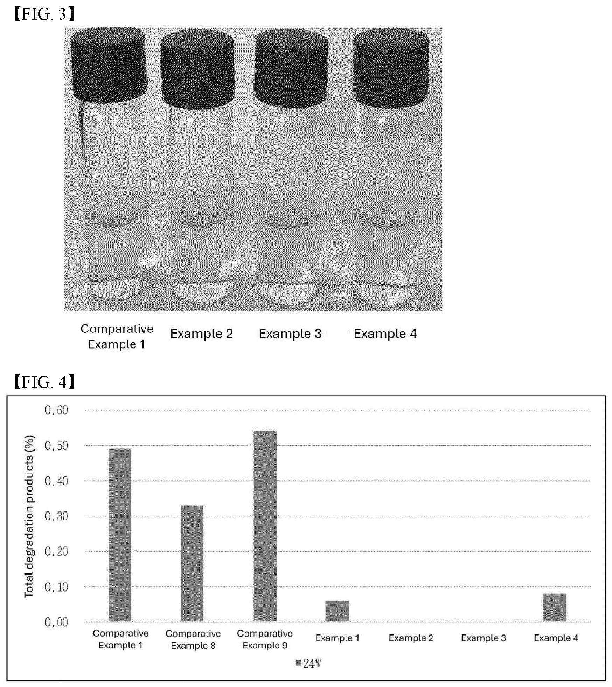

The results are shown in Tables 6 and 7 below and FIGS. 3 to 5.

FIG. 3 is a photograph showing the appearance of the ophthalmic compositions of Examples 2 to 4 and Comparative Example 1 after storage for 24 weeks at 40±2°C and 25% RH.

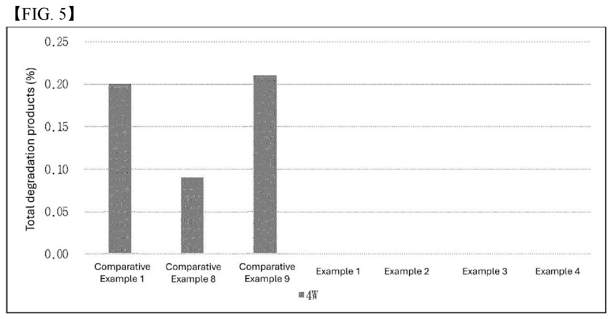

FIG. 4 is a graph showing the amount of total degradation products generated after storing the ophthalmic compositions of Examples 1 to 4 and Comparative Examples 1, 8, and 9 at 40±2°C and 25% RH for 24 weeks. FIG. 5 is a graph showing the amount of total degradation products generated after storing the ophthalmic compositions of Examples 1 to 4 and Comparative Examples 1, 8, and 9 at 50±2°C and 20% RH for 4 weeks.

**[Table 6]**

| | Storage conditions | Item | Initial (%) | 24 weeks (%) |
|---|---|---|---|---|
| Comparative Example 1 | 40±2°C, 25% RH | Individual degradation products | Not detected | 0.29 |
| | | Total degradation products | Not detected | 0.49 |
| Comparative Example 8 | 40±2°C, 25% RH | Individual degradation products | Not detected | 0.25 |
| | | Total degradation products | Not detected | 0.33 |
| Comparative Example 9 | 40±2°C 25% RH | Individual degradation products | Not detected | 0.33 |
| | | Total degradation products | Not detected | 0.54 |
| Example 1 | 40±2°C, 25% RH | Individual degradation products | Not detected | 0.06 |
| | | Total degradation products | Not detected | 0.06 |
| Example 2 | 40±2°C, 25% RH | Individual degradation products | Not detected | Not detected |
| | | Total degradation products | Not detected | Not detected |
| Example 3 | 40±2°C, 25% RH | Individual degradation products | Not detected | Not detected |
| | | Total degradation products | Not detected | Not detected |
| Example 4 | 40±2°C, 25% RH | Individual degradation products | Not detected | 0.08 |
| | | Total degradation products | Not detected | 0.08 |

**[Table 7]**

| | Storage conditions | Item | Initial (%) | 4 weeks (%) |
|---|---|---|---|---|
| Comparative Example 1 | 50±2°C, 20% RH | Individual degradation products | Not detected | 0.15 |
| | | Total degradation products | Not detected | 0.20 |
| Comparative Example 8 | 50±2°C, 20% RH | Individual degradation products | Not detected | 0.09 |
| | | Total degradation products | Not detected | 0.09 |
| Comparative Example 9 | 50±2°C, 20% RH | Individual degradation products | Not detected | 0.15 |
| | | Total degradation products | Not detected | 0.21 |
| Example 1 | 50±2°C, 20% RH | Individual degradation products | Not detected | Not detected |
| | | Total degradation products | Not detected | Not detected |
| Example 2 | 50±2°C, 20% RH | Individual degradation products | Not detected | Not detected |
| | | Total degradation products | Not detected | Not detected |
| Example 3 | 50±2°C, 20% RH | Individual degradation products | Not detected | Not detected |
| | | Total degradation products | Not detected | Not detected |
| Example 4 | 50±2°C, 20% RH | Individual degradation products | Not detected | Not detected |
| | | Total degradation products | Not detected | Not detected |

Through Tables 6 and 7 and FIGS. 4 and 5, it can be confirmed that the ophthalmic compositions of Examples 2 and 3, which contain propylene glycol as an anti-degradation agent but do not contain antioxidants, exhibit superior stability compared to Comparative Examples 1, 8, and 9, which do not contain propylene glycol as an anti-degradation agent, as well as compared to Example 1, which contains an antioxidant with propylene glycol as an anti-degradation agent.

Furthermore, Example 4 shows that stability decreases when the surfactant polysorbate 80 is additionally included along with propylene glycol as an anti-degradation agent.

Moreover, FIG. 3 confirms that the ophthalmic compositions of Examples 2 to 4 show no change in appearance after 24 weeks of storage at 40±2°C and 25% RH.

Although particular embodiments of the present invention have been shown and described, it will be understood by those skilled in the art that it is not intended to limit the present invention to the preferred embodiments, and it will be obvious to those skilled in the art that various changes and modifications may be made without departing from the spirit and scope of the invention.

The scope of the present invention, therefore, is to be defined by the appended claims and equivalents thereof.

## Claims

1. An ophthalmic composition comprising lifitegrast or a pharmaceutically acceptable salt thereof, and propylene glycol as an anti-degradation agent.

2. The ophthalmic composition according to claim 1, wherein the propylene glycol is included at a concentration of 0.2 to 5% (w/v).

3. The ophthalmic composition according to claim 1, further comprising one or more additives selected from a pH adjuster, a buffer, and an isotonic agent.

4. The ophthalmic composition according to claim 3, wherein the pH adjuster is one or more selected from the group consisting of lactic acid, hydrochloric acid, phosphoric acid, citric acid, acetic acid, sodium hydroxide, potassium hydroxide, sodium carbonate, and sodium bicarbonate.

5. The ophthalmic composition according to claim 3, wherein the buffer is one or more selected from the group consisting of phosphoric acid, phosphate, carbonate, bicarbonate, tris(hydroxymethyl)aminomethane (TRIS), lactic acid, lactate, citric acid, citrate, boric acid, borate, acetic acid, and acetate.

6. The ophthalmic composition according to claim 3, wherein the isotonic agent is one or more selected from the group consisting of glycerin, sodium chloride, potassium nitrate, potassium chloride, calcium chloride, magnesium chloride, trehalose, maltose, sucrose, mannitol, sorbitol, and boric acid.

7. The ophthalmic composition according to claim 1, wherein the ophthalmic composition is in the form of an aqueous solution.

8. The ophthalmic composition according to claim 1, wherein pH of the ophthalmic composition is within the range of 7 to 8.

9. The ophthalmic composition according to claim 1, wherein the ophthalmic composition contains no antioxidant.

10. The ophthalmic composition according to claim 9, wherein the antioxidant is one or more of dibutylhydroxytoluene, ascorbic acid, phenolic acid, sorbic acid, sodium bisulfite, sodium metabisulfite, acetylcysteine, sodium thiosulfate, ethylenediaminetetraacetic acid (EDTA), sodium nitrite, ascorbyl stearate, ascorbyl palmitate, alpha-thio-glycerol, erythorbic acid, cysteine hydrochloride, citric acid, tocopherol, tocopherol acetate, soy lecithin, sodium thioglycolate, butylated hydroxyanisole, propyl gallate, uric acid, melatonin, and thiourea.

11. The ophthalmic composition according to claim 1, wherein the ophthalmic composition is for treating dry eye syndrome.
